(19) **Europäisches Patentamt / European Patent Office / Office européen des brevets**

(11) **EP 4 610 493 B1**

(12) ## EUROPEAN PATENT SPECIFICATION

(45) Date of publication and mention
of the grant of the patent:
**24.06.2026 Bulletin 2026/26**

(21) Application number: **25159776.1**

(22) Date of filing: **24.02.2025**

(51) International Patent Classification (IPC):
*F04B 43/12* *(2006.01)* *A61M 60/109* *(2021.01)*
*A61M 60/247* *(2021.01)*

(52) Cooperative Patent Classification (CPC):
**F04B 43/12; A61M 1/3666; A61M 60/109;
A61M 60/279; A61M 60/38; A61M 60/835;
F04B 43/123**

(54) **PERISTALTIC PUMP AND EQUIPMENT FOR BLOOD TREATMENT**

PERISTALTISCHE PUMPE UND VORRICHTUNG ZUR BLUTBEHANDLUNG

POMPE PÉRISTALTIQUE ET ÉQUIPEMENT DE TRAITEMENT DU SANG

(84) Designated Contracting States:
**AL AT BE BG CH CY CZ DE DK EE ES FI FR GB
GR HR HU IE IS IT LI LT LU LV MC ME MK MT NL
NO PL PT RO RS SE SI SK SM TR**

(30) Priority: **29.02.2024 IT 202400004462**

(43) Date of publication of application:
**03.09.2025 Bulletin 2025/36**

(73) Proprietor: **Shenzhen Prunus Medical Co., Ltd.
Shenzhen (CN)**

(72) Inventor: **GAZZANO, Michele
20080 Albairate (MI) (IT)**

(74) Representative: **Mincone, Antimo
Viale Europa 101
50126 Firenze (IT)**

(56) References cited:
JP-A- 2001 304 131    SU-A1- 547 551
US-A- 3 020 846    US-A- 3 674 383
US-A- 4 522 571    US-A- 4 530 647

**Description**

The present invention relates to a peristaltic pump and equipment for the treatment of blood.

As will be more fully described below, the peristaltic pump object of the present invention is coupled to a removable, preferably disposable kit that is an integral part of the pump and houses the under-pump tube and any additional components for both control and eventual treatment of the pumped liquid.

The invention fits into the medical field and, in particular, into the treatment of blood in extracorporeal circulation, a field in which the following aspects are of relevant importance: the reduction of trauma (hemolysis), the possibility of rapid circuit replacements, the guarantee of asepsis, the reduction of risk related to possible human errors, and the simplicity and safety of execution.

This will not prevent their use from being extended to other, equally critical areas such as food processing, the pharmaceutical and chemical industries, etc.

Common peristaltic pumps usually consist of an outer cylindrical stator and an inner cylindrical rotor coaxial to the stator. The rotor generally comprises two or more skids or rollers and is driven by a motor connected to it. The motor sets the internal rotor in rotation, which drives in its circular motion the rollers (or skids) that describe a cylindrical orbit. A portion of the tube or "under-pump tube" (in fact, this is how the segment of elastic tube subjected to the peristaltic action of the pump is called in technical jargon) is manually placed between the space between the inner surface of the stator, which acts as the reaction plane, and the circumference described by the pressing elements (skids or rollers) that rotate integral with the rotor, arranged along the latter's outer surface.

In peristaltic pumps of the known type, the space intended to accommodate the under-pump tube is designed so that it is equal to the sum of the wall thickness of the tube to be compressed (small variations more or less are chosen according to the type of pipe or fluid to be pumped). The rollers compress the tube by progressively occluding it against the stator in their rotary motion. The compressed pipe generates flow by change in its internal volume. In the section of tube arranged upstream of the compression carried out by the rotor (i.e., on the portion already affected by the compression of the pressure rollers), the tube regains its original shape due to its elastic memory by sucking in the fluid to be pumped. The cyclic, one-way repetition of this action enables pumping.

Peristaltic pumps of the known type are not always able to provide high quality standards with regard to the above-mentioned aspects, i.e., with respect to the reduction of trauma, the possibility of rapid circuit replacements, the reduction of manual interventions for the replacement of the under-pump , the guarantee of asepsis, the reduction of risk related to possible human errors, and the simplicity and safety of execution.

An initial solution to the problems of known art was provided by WO/2018/146541, a document that relates to a pump of the type comprising a stator supporting a portion of the pipe intended for the passage of the fluid to be pumped and a single pressor element capable of exerting pressure on the pipe to result in a cyclic constriction of the latter for the movement of the fluid within it.

The pump described in WO/2018/146541 is provided with a single pressor element equipped with means of motorization for rotation about its axis designed to cause it to travel a circular trajectory when in contact with the pipe resulting in a different value of pipe crush along the trajectory followed by the pressor element. A further pump is known from JP2001 304131.

The present invention seeks to further improve the characteristics of a pump intended for medical use for handling blood in general and, in a specific application case, for pumping venous blood taken from a catheter through a component suitable for $CO_2$ removal consisting of an oxygenator by defining the equipment covered by the present invention.

Advantageously, the pump that is the subject of this patent application is embedded in equipment that also includes other components such as pressure gauges to control pressures (suction, prefilter, and return), safety shutoff devices (or "clamps") that intervene in case of alarms, air bubble sensors, as well as the oxygenator (or other component that performs the necessary function for the desired treatment).

Among the purposes of the present invention is, therefore, to provide very high standards for the safety of treatment (i.e., relative to the patients and operators involved) and for the substantial absence of damage (hemolysis) suffered by the treated blood due to the action of a single truncated-cone presser, the structural and functional characteristics of the pump and the equipment in question.

In addition to the advantages related to the substantial absence of blood damage, the remarkable simplicity of assembly of the component parts of the present invention (i.e., the possibility of assembly of the removable, disposable holder containing the under-pump by coupling it directly to the presser without the need for auxiliary mechanisms that perform translational movements for proper positioning) can be cited among the additional advantages of the present invention. This results in increased simplicity and safety of use; moreover, even at the construction level, the realization of the pump is simplified due to the innovative conformation of the rotor and stator (truncated cone), which results in an advantage related to the characteristic of the peculiar flow produced by the truncated cone presser on a under-pump tube arranged on a helical path with a variable diameter. Such flow is particularly suitable for reducing hemolysis generated by shear stress (shear stress). A further advantage relates to the possibility of stator molding with a reduction in the number of molds and corresponding simplification of design and lowering of costs.

The advantages and features of the invention will be more evident from the description that follows which refers to the accompanying drawings, provided as examples and not limitation, in which:

- Fig.1 is a schematic perspective view of an example of an embodiment of an apparatus formed by a pump in accordance with the present invention associated with a device for removing $CO_2$ from blood mounted on the removable kit containing the stator and complete with the accessory elements.
- Fig.2 is a top view of the example shown in Fig.1.
- Fig.3 is a top view of the example shown in Fig.1 according to a plane parallel to the plane presented above by the equipment.
- Fig.4 is a schematic perspective view of the example in Fig.1, in which the equipment has been deprived of a part supporting the stator, under-pump tube and other component parts that are part of the removable and preferably disposable assembly.
- Figs. 5, 6 and 7 are for the part supporting the stator, under-pump tube and other preferably disposable component parts, shown in a perspective view (Fig.5), a plan view from above (Fig.6) and a plan view from below (Fig.7), respectively.
- Figs. 8 and 9 show a possible example of how to arrange the under-pump tube inside the stator shown in a side view (Fig.8) and a plan view from above (Fig.9), respectively; and
- Fig.10 is a sectional view according to the X-X line of Fig.3.
- Figs. 11 and 12 are enlarged details of Fig. 10 in different scales.
- Fig. 13 is a schematic regarding the arrangement of the presser and stator of the pump subject of the present invention with special reference to the sizing and positioning of the two elements.

The following definitions will be used in this description to identify the components. The term "under-pump pipe" or "under-pump tube" or also "circuit-tube" means the portion of tube that passes through the pump and on which the pump itself exerts the thrust suitable for determining its motion.

"Presser" means the element (with a truncated conformation in the present solution) that is in direct contact with the under-pump tube and it is placed and configured to press the same under-pump tube in order to generate a blood flow inside the latter. "Rotor" means the rotating base that supports the shaft, inclined with respect to the axis of the rotor itself, around which the presser rotates and which, due to the rotation of the rotor around its own vertical axis, causes the presser to describe a truncated orbit.

"Stator" means the element with a conical cavity that properly couples with the presser to form the pump and forms an integral part of it by providing a reaction surface against which the presser compresses the under-pump tube, generating blood flow according to the principle of peristalsis.

"Stator base" is the support integral with the stator that allows its proper alignment with the pump and its attachment (in the preferred realization such an assembly consisting of stator and stator base is made by injection molding of rigid and transparent plastic material (e.g., polycarbonate or rigid PVC) to which the necessary tubing connecting the parts and the patient are properly connected; for uses other than medical, realization in reusable material can be expected);

"Pump base" is the plane to which the pump is connected, and which contains the elements to properly align the stator base with the pump. The "stator base," "stator," and "under-pump tube" elements are part of the removable and preferably disposable assembly to which the oxygenator and tubing (in turn connected to the catheter and patient) are connected.

With reference to the drawings in the attached figures, a peristaltic pump (100) made in accordance with the present invention is of the type comprising: a stator (1) supporting a portion of a pipe or under-pump tube (2) intended for the passage of a fluid to be pumped; a presser (3) suitable for exerting pressure on said under-pump tube (2) to bring about a cyclic shrinkage of said under-pump tube (2) for the movement of the fluid therein; and a base (8) supporting the pump (also called a pump base) provided with locking means (20A, 20B) for a body defining the stator (1) integral with its own base, indicated by reference (6). Specifically, the pump (100) is composed of its main components: a base unit (101) and a stator unit (102), which, as mentioned earlier, can advantageously be disposable when the use of the pump makes it preferable such as, for example, in use as a blood processing equipment.

In the nonlimiting example shown in the drawings, the apparatus (200) includes a pump (100) and an oxygenator (103) that is associated with the stator unit (102), which is of the disposable type. In other possible embodiments of the invention, the apparatus (200) may include a pump (100) and another device other than the oxygenator.

In the preferred embodiment depicted in the drawings, the two components (101) and (102) are joined by creating a depression that results in the stable union of the two parts, as will be described below.

The stator (1) consists of a hollow body defining within it a substantially truncatedconical cavity and provided with an internal groove (17) that extends for at least 360° and accommodates the under-pump or circuit-tube (2), keeping it properly positioned and exposed to the inside of the stator itself (1).

Specifically, in the configuration shown in the drawings (but not limiting), the inner truncated conical cavity of the stator (1) includes a conical surface that has an opening (A1) equal to 60° in cross section. On the said conical surface is formed the internal groove (17) that accommodates the under-pump (2).

The presser (3) of the pump (100) has a substantially truncated conformation and in the nonlimiting configuration shown in the drawings has an opening (A3) equal to 45°. The pressor element is mounted by means of bearings (31) on its shaft (30), which is inclined by 7.5° in the example shown with respect to the axis of rotation of the rotor (90) in order to obtain a correct direction of the forces compressing the under-pump tube, which are thus perpendicular to the surface of the presser (3) and the stator (1) at the point of contact with the under-pump tube (2). Such perpendicularity ensures that the position of the under-pump tube is properly maintained within the stator by eliminating force components that would tend to dislocate it, compromising proper operation.

Specifically, the inclination of the shaft (30A) of the presser (3) with respect to the axis (90) of rotation of the rotor (9) is an angle equal to half the difference between the angle of opening of the truncated cone defined by the stator and the angle of opening of the truncated cone defining the presser; in the case illustrated, this value is equal to half the difference between the value of (A1) and the value of (A3), i.e. (60° - 45°)/2 = 7.5°.

In other words, the conical surface defining the pressor engagement surface (3) is the conical surface of a right cone whose vertex (A) belongs simultaneously to the axis of the truncated cone inside the stator (90), the axis of the rotor (90) and the axis of the truncated cone of the pressor itself (30A). Thus, the ratio between the diameter of any section perpendicular to its own axis obtained from the truncated cone of the pressor and the truncated cone of the stator when both sections pass through the same contact point between the pressor and stator is constant.

In this way, the linear velocity of the rolling of the presser on the compressed surface of the under-pump will always be the same at each point of contact, avoiding axial components that would dislocate the under-pump from its correct position.

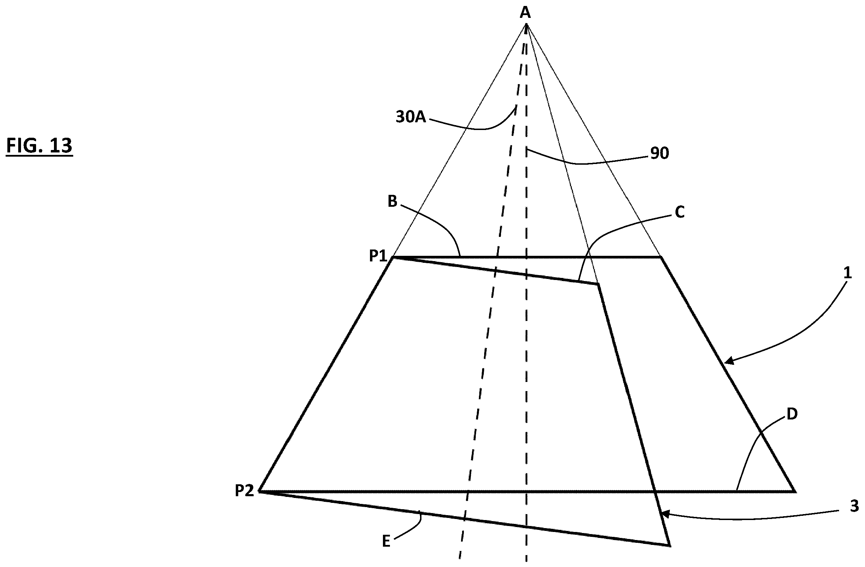

Fig. 13 depicts the "geometry" of the pump in question; the drawing shows the axis (90) of the stator (1) (and rotor 9), the axis (30A) of the presser (3) and the point (A) that is defined by the intersection of said axes (90, 30A) as well as the extensions of the apothems of the truncated cones that define the presser (3) and stator (1).

In Fig.13, in other words, the vertices of the cones describing the stator and the presser originate from the same point (A), which is simultaneously the origin of the respective axes (90, 30A). In the above configuration, the stator (1) and the presser (3) are represented by the corresponding sections perpendicular to their respective axes; reference (B) corresponds to the minor base of the stator (1), reference (C) corresponds to the minor base of the presser (3), reference (D) corresponds to the major base of the stator (1), reference (E) corresponds to the major base of the presser (3), and references (P1) and (P2) represent two points of engagement between the presser (3) and the stator (1).

Experimentally, it was possible to prove that the following relationship exists:

$$B:C=D:E$$

for any reciprocal positioning between the presser and stator along the orbit defined by the presser. In practice, at any point of engagement along the orbit, the above relationship is valid.

The presser (3) is idler and is drawn into its orbit describing a truncated conical surface by the rotation of the rotor (9) which, as hereinafter described, is connected to the motor means (106) of the pump (100).

In accordance with the present invention, a peristaltic pump (100) is, therefore, of the type comprising, in its most general structure the following components:

- a portion of tube or under-pump tube (2) intended for the passage of a fluid to be pumped, lying between an inlet (22) and an outlet (23, and on which to exert a pressure suitable for generating a direct flow of the fluid from the inlet to the outlet;
- a stator (1) which is provided with an internal cavity bounded by corresponding inner walls (17) against which said portion of the tube (2) is pressed;
- means (17) to keep the portion of tube (2) in the correct position relative to said stator (1) (the means to keep the portion of tube 2 in the correct position are formed by the inner walls 17 of the stator 1 which accommodate the portion of tube 2);
- a presser (3) that is moved cyclically along a trajectory to engage said portion of the tube (2) by exerting pressure on it through a corresponding engagement surface.
- means of motorization of said presser (3) comprising a motorized base or rotor (9) that is rotated about an axis (90) and that in its rotation is integral with the presser (3), which is free to rotate about its axis (30A) with respect to said motorized base or rotor (9).

Advantageously, pump (100) is characterized in that:

- the inner walls (17) of said stator (1) are developed along a conical surface having a first opening (A1) and an axis coincident with the axis of rotation (90) of said rotor (9) (the inner walls form an internal groove that, as better specified below, accommodates the tube 2);
- the engagement surface of the presser (3) is a conical surface having an opening (A3) that is different and smaller than the first opening (A1);
- the presser (3) is supported by a shaft (30) that has the same axis (30A) of the presser (3) and that is not coincident with the axis of rotation (90) of the rotor (9) to which it is integral but still passing through point A.

In other words, at the inner of the presser (3) there is the shaft (30) that supports the presser (3) has the same axis (30A) of the presser. The shaft (30) is integral to the rotor (9) and the axis (30A) of the shaft (30) doesn't coincide with the axis (90) of the rotor (9).

The inclination of said axis (30A) with respect to the axis (9) is equal to half the difference between the angle of the opening (A1) of the inner cone of the stator (1) and the angle (A3) of the opening of the cone of the presser (3).

As visible in the drawings, the presser (3) is preferably provided with an upper appendage (16) formed by a cylindrical body inclined, that is, presenting an axis not coincident with that (30A) of the presser (3) but coincident with that (90) of the rotor (9); the stator (1) is correspondingly provided with an internal cavity (15) conformed and arranged to accommodate said upper appendage (16) allowing the latter to rotate within said internal cavity (due to the presence of anti-friction elements, in the illustration ball bearings) (15).

Therefore, the conformation of the body of the presser (3), which has been found to be particularly effective and advantageous in the experiments carried out, is determined by the association in a single body of a truncated cone (forming the lower part of the presser 3) surmounted by a cylinder forming the upper appendage (16) provided with a ball bearing or other means of reducing friction with respect to the housing (15) in which it is inserted.

In practice, the shaft (30) of the presser (3) is surmounted by an appendage integral with the shaft itself (30) and angled so as to be perpendicular to the axis (90). Said appendage has a cylindrical protrusion with a circular base coaxial to the axis (90) on which cylindrical protrusion is mounted a bearing (or other anti-friction element capable of permitting its rotation within the internal cavity (15) of the stator.

This arrangement makes it possible to unload forces resulting from the pressure action of the presser against the under-pump tube that might otherwise result in undesirable oscillations of the stator about its axis (90).

The rotor (9) is keyed to a shaft (91) presented at the output of a motor (106); the motor (106) and the connection with its driven shaft (91) are shown schematically in the drawings.

The rotor (9) is placed inside a base (8) with respect to which it is rotatable, unlike the stator (1), which, in contrast, is integral to the base (8) when the pump (100) is in use configuration.

In addition, the rotor (9) consists of a lower portion (9B) directly keyed to the shaft (91) from which it receives motion, and an upper portion (9A) that has a sloping upper surface from which it rises perpendicularly an inner core (33) of the presser (3), which will be described later.

In practice, the inclination between the inner core (33), which defines the axis (30A) of rotation of the presser (3), with respect to the bottom surface of the rotor (9), which is horizontal, determines the truncated orbit followed by the presser and the correct inclination required for the presser to roll without creeping on the surface of the under-pump (3).

The two portions (9A) and (9B) of the rotor (9) are made integral with each other by screws (93). In the attached figures, the rotor can be identified with (9) in its entirety or with (9A) and (9B) to differentiate its upper and lower portions.

The base (8) is connected to a fixed flange (80), arranged inferiorly, and crossed by the shaft (91). The fixed flange (80) is attached to the base (8) by a series of screws (81) that pass through a hollow cylindrical crown (82) inside of which is housed the lower portion (9B) of the rotor (9) with the interposition of bearings (92) that make the rotor (9) rotatable with respect to the base (8) and its fixed flange (80).

The fixed flange (80) has a central cylindrical portion (80A) that is crossed by the central part of the lower portion (9B) of the rotor that is integral with the shaft (91). A lip seal (109) is provided between the central part of the lower portion (9B) and said cylindrical central portion (80A) to ensure sealing.

In other words, the means of motorizing the presser element (3) may include a motorized base or rotor (9) integral to the presser shaft (30) on which the presser (3) is free to rotate about its axis (30A) relative to said motorized base or rotor (9). Specifically, with reference to the example shown in the drawings, the rotor (9) is provided with a lower (9B) connecting portion that is keyed to the shaft (91) of a gear motor (106). The lower portion (9B) of the rotor is attached integral to the upper portion (9A) of the rotor (9) by means of one or more retaining pins (93). Friction-reducing means are provided around the rotor (9), which may consist, for example, of ball bearings (92); around the shaft (91) there are one or more seal(s) (109) that allow the shaft to rotate with respect to the crankcase defined by the central cylindrical portion (80A) and that, in essence, are not permeable to air.

The latter condition occurs because appropriate atmospheric depression is provided. In other words, a casing is defined that consists of the fixed flange (80) that has a planar development and is overlaid by the hollow cylindrical part (80A) within which the lower portion (9B) of the rotor (9) is contained. On the cylindrical part (80A) of the casing is attached integral the base (8) of the pump, which on the top is provided with a plate (7) made, for example, of elastomeric material. Specifically, the plate (7) is inserted inside the frame (20A) that is part of the locking and alignment means (20) of the base (8). The clamping and alignment means (20) comprise a plate (20B) defining the stator base (1) and provided with a second frame (21) complementarily conformed with respect to the first frame (20A).

Again, with reference to the attached drawings, the axis of the upper appendage (16) of the presser is coaxial to the axis (90) of the rotor (9) and stator (1).

In addition, as indicated above, the presser (3) includes a shaft (30), internally placed, that is connected to the rotor (9) and integral to the same rotor (9), having an outer truncated cone body (32) that defines the aforementioned engagement surface of the presser (3). Bearings (31) are provided between the inner shaft (30) and the truncated cone body (32) designed to make the truncated cone body (32) rotate in neutral with respect to the inner shaft (30).

Specifically, the shaft (30) of the presser (3) is a hollow cylinder coaxial to said inner core (33) (which is integral to the rotor as mentioned earlier), and by an outer bushing (34), interposed between the inner core (33) and the bearings (31).

In the shaft (30), between the inner core (33) and the outer bushing (34), there are elastic connecting means (35, 36, 37) configured to allow radial clearance between the two parts. In practice, the inner core (33) is provided with two feed-through seats (38); in the inner face of the outer bushing (34) are stably housed two interference pins (35) that instead slide freely through said feed-through seats (38). These pins allow a modest radial sliding of the shaft (30) of the presser (3) while preventing its rotation with respect to the inner shaft (33); this movement makes it possible to compensate for the unavoidable tolerances in the dimensions of the under-pump tube (2).

Coaxially to these pins but from the opposite side are attached springs (37) that in their free end support relative thrust elements (36) that, coming out of the seats (38) go to engage the inner surface of the bushing (34) from the opposite side to that of the interference pin attachment (35).

In this way, slight radial displacements are allowed between the central core (33) that is directly connected to the rotor and the outer bushing (34) that is connected via the bearings (31) to the truncated cone body (32) that defines the engagement surface with the under-pump (2).

In the operation of the pump (100), as mentioned earlier, the presser (3) is led into rotation by the rotor (9) that supports it by defining a truncated orbit that leads to engaging the under-pump with the outer surface of the truncated casing (32). In the rotation of the presser (3) its upper appendage (16) which is provided with bearings rotates within the housing (15) of the stator (1).

The special handling determined by the rotation around the axis (30A) of the presser (3) and the rotation of the appendage (16) around the axis (90) contributes to the optimal interaction between the engagement surface (32) of the presser (3) and the under-pump (2).

Among the advantageous features of the invention can be mentioned that the following can be listed:

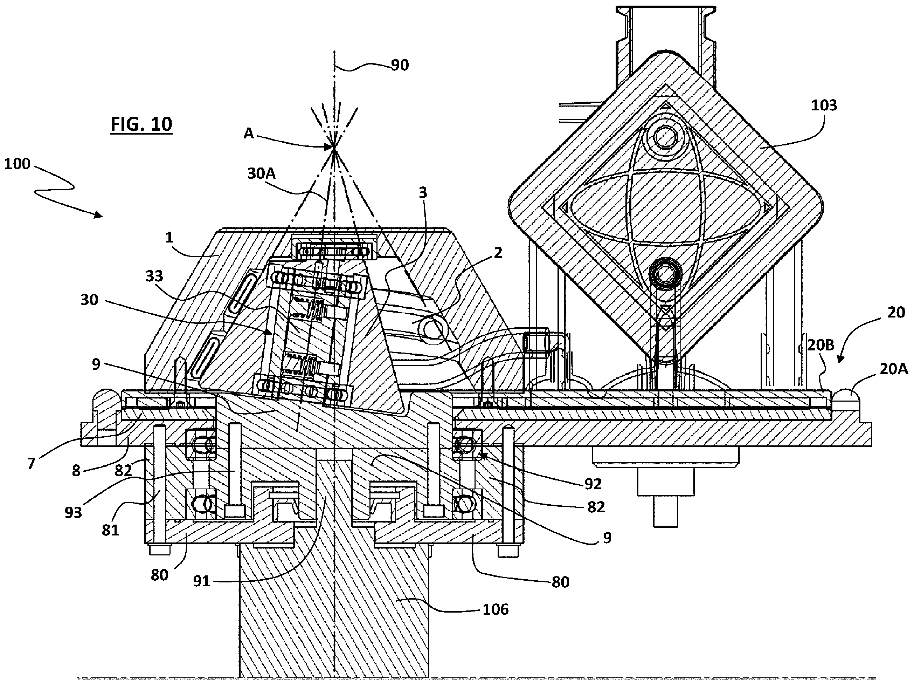

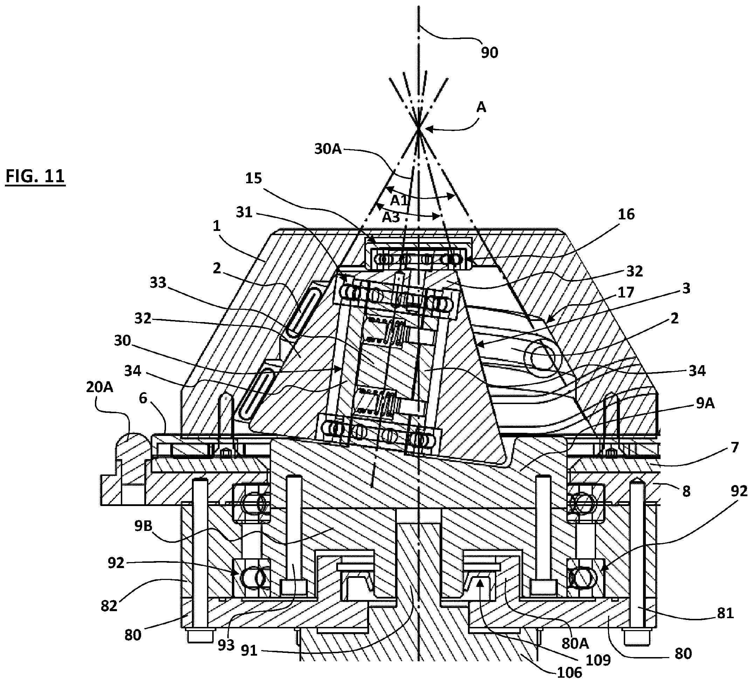

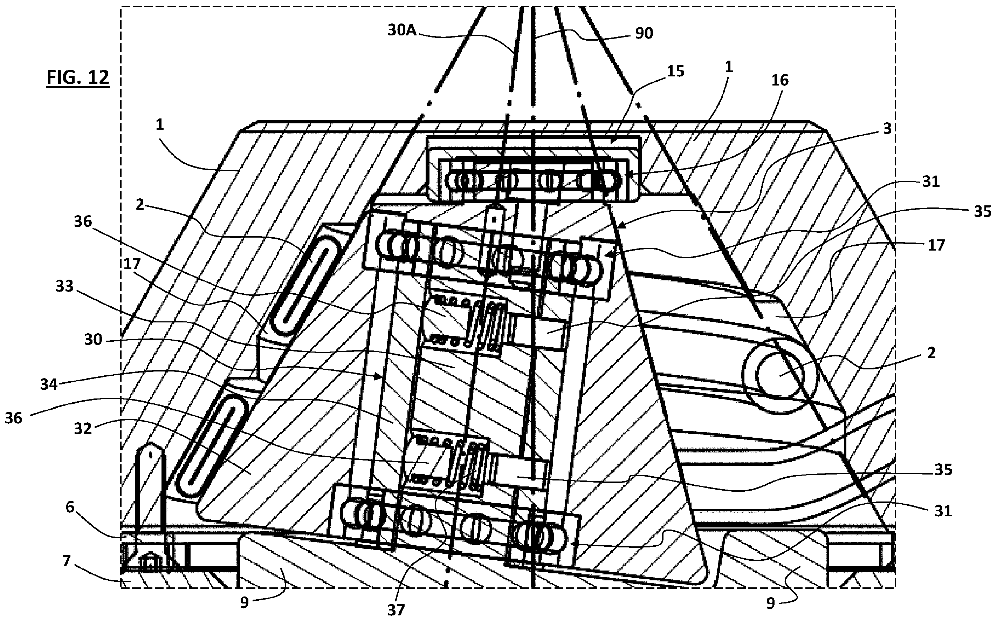

- the inner walls of the said stator (1) form with the axis (90) thereof an angle between 15° and 60°; (30° in the preferred realization and in Figs. 10; 11;12, where said angle is indicated with the reference A1)
- the outer walls of the truncated cone body of said presser (3) form with the axis (30A) of the presser (3) an angle equal to half the difference of the opening (indicated by the reference A1) of the hollow truncated cone inside the stator (1) and the opening angle of the truncated cone formed by the presser (3) (indicated by the reference A3), so that the orbit described by the presser (3) during the rotation of the rotor (9) about the axis (90) is concentric and equidistant from the internal walls of the stator (1) (walls that define a hollow truncated cone cavity) and maintains at each point the same linear velocity relationship with it.

The pump (100) is, therefore, composed of a base unit (101) and a stator unit (102) in which said base unit (101) comprises at least the presser (3) and the rotor (5) and said stator unit (102) comprises at least the stator (1), the tube portion (2), there being provided a base (8) arranged around said rotor (5) not integral with said rotor (5) in rotation and provided with a plate (7) of material suitable for defining a pneumatic seal, there being provided means for determining a lower pressure for associating said base (8) and said stator unit (102) as a seal, resulting in a stable association between said base unit (101) and said stator unit (102).

The aforementioned means of aiding in the proper positioning of the under-pump tube or portion of tube (2) to the stator (1) may include the internal groove formed by the internal walls (17) of the stator (1), which is developed according to a conical helix, with pitch (17A) proportional to the value (18) of the width of said portion of tube (2) when occluded. In practice, in the preferable single-use configuration of the stator unit (102), the under-pump or tubing portion (2) comes already assembled to the stator (1) and the other single-use components, greatly simplifying blood processing operations, both from the point of view of safety and efficiency.

In this way, it is possible to provide an apparatus (200) for venous blood treatment that includes a pump (100) made as described above and a disposable kit including an oxygenator device (103). The tubing portion (2) has an inlet (22) and an outlet (23), and the oxygenator device (103) has an inlet (24) and an outlet (25); the outlet (23) of the tube portion (2) is connected to the inlet (24) of the oxygenator device (103) via in connection made on the underside of the stator base, while the inlet (22) of the tube portion (2) is connected via in connection made on the underside of the stator base to the connector (104); similarly, the outlet (25) of the oxygenator device (103) via in connection made on the underside of the stator base is connected to the connector (105). From these connectors branch off the usual tubing (not shown) for connection to the patient for the input of blood to be treated (104) and the return of treated blood (105), respectively.

As is well known, an oxygenator device (103) is a device designed to carry out gaseous exchange between air and patient, analogous to what takes place inside the lungs; in this particular implementation, the goal of CO2 removal is rather pursued although an oxygenation component is, however, present. Advantageously, in the case of equipment with an oxygenator, the pump (100) is provided with means of motorization (106) suitable for determining a flow between 50 ml/min and 2,000 ml/min. although, in order to reduce the invasiveness of the treatment and side effects it will be preferable to limit the flow around 500 ml/min.

As indicated above, the contact between the base (20B) of the stator (1) and the base (8) of the pump is made in such a way as to define a pneumatic seal through contact with the plate (7) made of elastomeric (silicone) material that acts as a seal between the stator base (6) and the pump base (8). Means (not shown) are provided to determine a lower pressure (or vacuum) to associate the pump base (8), the stator base (6), and the stator itself (1) with a seal.

Means to bring about a vacuum may include a pump capable of reducing the pressure inside the chamber defined by the stator (1) and its base (20B) resting on the plate (7).

Such a vacuum pump is commonly available (e.g., a diaphragm pump) is not described in detail or illustrated in the drawings but may be of a similar type to that described in European Patent No.EP2575926B1. The vacuum generated by the pump allows pressure from the outside to be obtained on the entire surface of the stator base (20B) and the stator itself (1) so as to ensure proper clamping of the stator on the machine base.

This pressure is amply sufficient to counteract the thrust of the pump against the under-pump tube. The vacuum within the truncated cone cavity that houses the under-pump tube is also functional and useful in helping the pipe itself quickly recover its original shape as a result of the pressure exerted by the presser, thereby increasing the efficiency and consistency of the pumping effect over time.

In operation, experimental verifications have shown that when the stator (1) is properly mounted on the base (8), the appendage (16) of the pump fits into the cavity (15) and allows the forces transmitted by the presser against the under-pump tube (2) during crushing and thus against the stator itself to be effectively discharged. This solution avoids unwanted oscillations of the stator (1) that would compromise the effectiveness of the pumping action and the life of the stator itself.

Experimentally, it has been found that the described conformation allows an optimization of the interactions between the presser (3) and the under-pump tube (2) housed in the inner walls (17) of the stator (1).

From what has been described so far, the advantages of the present invention appear obvious, which are determined by its special features that will be described again below.

The presser (3) consists of a truncated cone-shaped element that, in the non-binding example in the drawings, is generated by the rotation of a line inclined 22.5° from the cone axis; smaller and larger apertures appear acceptable.

The presser (3) is mounted on a shaft (30) integral with the rotor (9). In particular, as indicated above, the shaft (30) is mounted on the inner shaft (33) that is integral to the rotor (9). This axis is inclined in the example shown by 15° to the axis of rotation of the rotor (5), but this value is not binding and different inclinations are possible; in any case the inclination of said axis shall be equal to half the difference between the angle at the vertex of the cone describing the opening of the stator where the under-pump tube resides and the angle at the vertex of the cone describing the opening of the presser, this angle of the presser will necessarily be less than the angle of the stator

The surface of the presser (3), orbiting the axis (5) of the rotor, describes a truncated cone surface with an axis coincident with the axis (5) of rotation of the rotor.

The stator (1) has a truncated-cone cavity with the same inclination as the surface described by the presser (3) orbiting the axis (90) of the rotor (30° in the example) and coaxial to it.

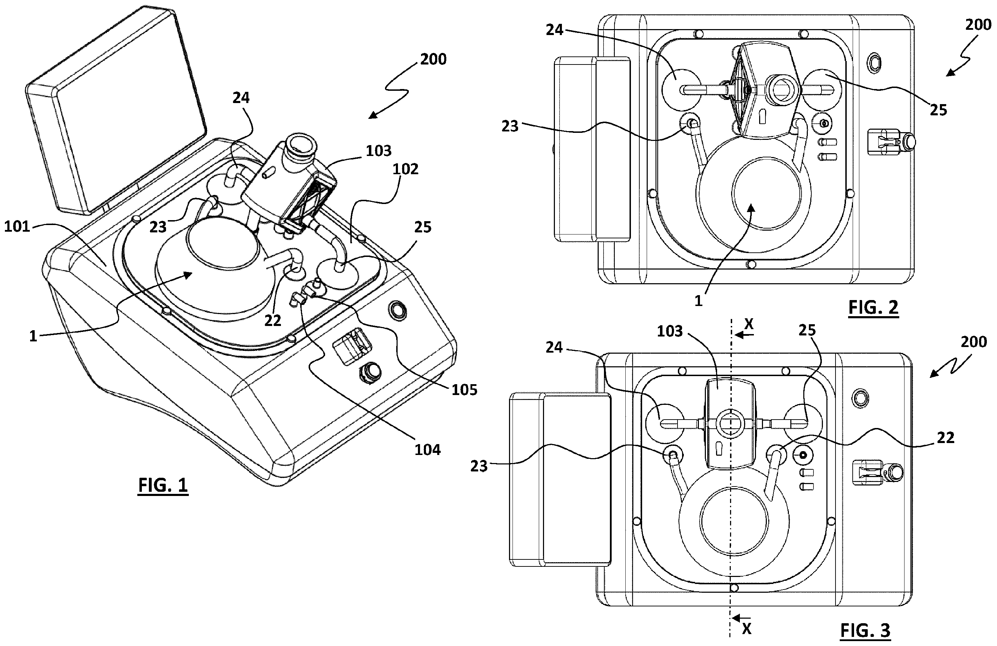

The stator has within said cavity, appropriate reliefs (the groove 17) designed to keep the under-pump tube (2) in the right position while the same is subjected to the compression generated by the presser (3) during its orbit. In Fig. 1, the under-pump tube (2) in the section of (Fig. 1) can be seen in "closed" or compressed configuration to the left of the rotor axis and in "open" or uncompressed configuration in the section to the right of the rotor axis.

The reliefs forming to groove (17) are characterized by a helical (in 25 particular conical) development whose pitch (30) is proportional to the length of the occluded under-pump pipe section (2).

Reliefs (17) follow the inner contour of the truncated cavity to allow the presser (3) to fully compress the tube until it causes complete occlusion for at least 300° of the orbit itself.

With the same helix pitch but different radius, the profiles will gradually move away from the axis of the stator cone to allow the inlet and outlet sections of the under-pump (2) to progressively come into contact with the tube thus allowing a gradual transition from the condition of totally open tube to that of totally occluded tube (vice versa in the outlet section); this solution is intended to minimize the traumatic effects of the pressor (3) on the solid components of the blood (red blood cells, platelets, etc.) greatly reducing hemolytic damage.

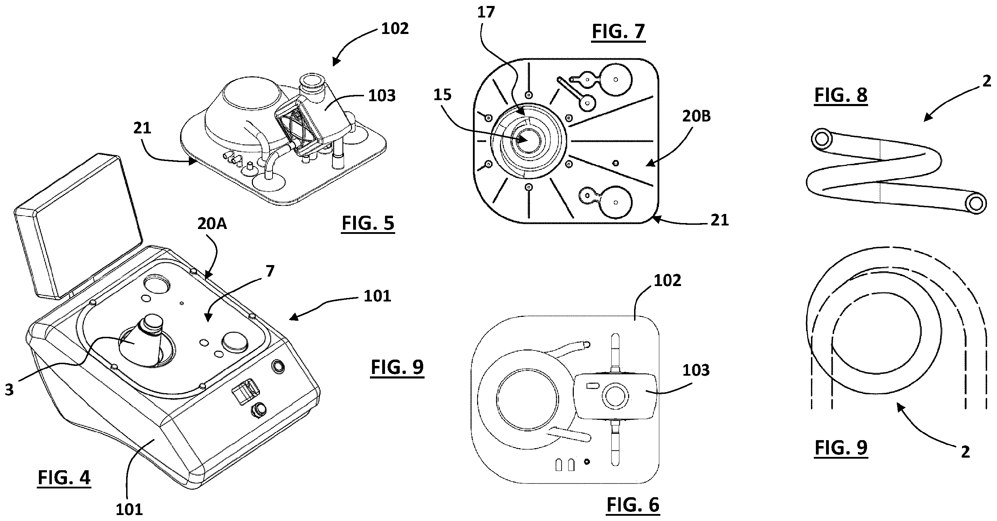

In the example shown (see in particular Figs. 8 and 9) the angle of the inlet and outlet sections of the pipe (shown in discontinuous section in Fig.9) cover an arc of 90 degrees although this value can appropriately be greater or lesser for a total development of the under-pump pipe greater than 360 degrees (in the example shown 540 degrees).

Experimentally, it has been ascertained that the preferable configuration condition is one in which the axes of the conical cavity of the stator (1) and rotor (9) are coincident, and the axis (30A) of rotation of the presser (3) mounted on the stator support (1) passes through a point (A) belonging to the extension of the aforementioned common axis.

This ensures that the direction of the vector of the compressive force generated by the presser (3) on the tube (2) (and on the stator 1) is at each point perpendicular to the tangent at that point on the stator surface (1), to avoid different components on the tube itself that would tend to displace it from its default position.

There is appropriately present on the presser (4) an extension concentric to the axis of rotation of the rotor positioned at the distal end of the holder for the presser which, by inserting into a compatible hole present on the stator (also concentric to the axis of rotation of the rotor), helps to unload the force necessary to compress the under-pump exerted by the presser on the stator from the rotor itself. This reduces the stress that the stator has to bear by allowing a lighter construction. The rest of the force will be borne by the attachment of the stator to its own base and from it to appropriate guides on the base of the pump (101) and stator (102).

The assembly of the stator (1) and its base (20B) define, in practice, a disposable kit and can be provided in appropriate disposable plastic material, preferably transparent to allow control of the operation and integrity of the under-pump tube (2) (PVC or Polycarbonate, to name a few).

Locking in place of the aforementioned disposable kit (102) can advantageously be achieved by means of a partial atmospheric vacuum. The partial vacuum has, in addition to the possibility of firmly joining the pump and its base with the above-mentioned assembly, the advantage of facilitating the filling of the tube upstream of the pump action, of requiring a under-pump tube (2) with a lower wall thickness (since the force that allows the tube to recover its resting shape does not depend solely on its elastic memory but also on the atmospheric vacuum outside it) to the advantage of a higher flow rate with the same external diameter of the under-pump and rotation speed, and also to a greater constancy in pump performance over time. Another fundamental advantage of the realization of the present conical pump (100) 10 lies in the fact that it can be easily coupled with its own disposable kit (101, 102) without requiring manipulation or the use of mechanical means of translation and fixation; it will be sufficient to place the kit (102) on the pump base (101) bringing it closer along the direction of the axis (90) aligning it along the locking means (20A) and (20B), the action of the vacuum will provide for the correct positioning of the kit and hold it in that position.

A further advantage lies in the fact that by rotating the rotor (9) in the appropriate direction, i.e., that which presses the tube (2) according to the direction that goes toward the increasing radii of the spiral in which it is forced by its housing (represented by the internal groove formed on the walls 17) in the stator (1), the volume of blood pumped shifts toward larger tube volumes (given by the increasing radius of curvature at the same angle covered).

This behavior negates overpressures within the tube itself that can give rise to hemolytic "shear stress" trauma with advantages for the atraumatic behavior of the pump itself.

The apparatus (200), pump (100), oxygenator (103) as well as all circuits and connecting parts will be suitably equipped with components such as pressure gauges to monitor pressures (suction, prefilter and return), safety shutoff devices (or "clamps") that intervene in case of alarms, air bubble sensors, etc. (not shown).

In general terms, as is evident from the description of the examples shown above, the present invention consists of a peristaltic pump comprising: a portion of a tube or under-pump (2) intended for the passage of a fluid to be pumped, included between an inlet and an outlet, and on which a pressure suitable for generating a flow of the fluid directed from the inlet to the outlet is exerted; a stator (1) which is provided with an internal cavity bounded by corresponding walls against which said portion of the tube is pressed; means (17) for attaching said portion of the tube (2) to said stator (1); a presser (3) which is moved cyclically along a trajectory to engage said portion of the tube (2) by exerting pressure on it by means of a corresponding engagement surface; means of motorizing said presser element (3) comprising a motorized base or rotor (9) which is rotated about an axis (90) and which in its own rotation is integral with the presser (3), which is free to rotate about its axis (30) with respect to said motorized base or rotor (9).

The pump (100) in question is characterized by the fact that: the inner walls of said stator (1) are developed along a conical surface having a first aperture (A1) and an axis coincident with the axis of rotation (90) of said rotor (9); the engagement surface of the presser (3) is a conical surface having a second aperture (A3) different from and lower than said first aperture (A1); the presser (3) is supported by an inner shaft (30) that has the same axis (30A) of the presser (3) and that is not coincident with the axis of rotation (90) of the rotor (9) to which it is integral but inclined with respect to said axis.

It is also evident from the above description that a pump in accordance with the present invention may have one or more of the following features, even combined with each other:

- the axis (30A) of the presser (3) is inclined to the axis (90) of the rotor (9) by an angle equal to half the difference between the said first opening angle (A1) of the stator and the said second opening angle (A3) of the presser.

- the axis of rotation (90) of the rotor (9), the axis of the conical surface of the inner walls of the stator (90), the axis of the presser (30A) and the axis of the conical surface to which the presser surface (30A) belongs are all intersecting at a common point (A).
- to the distal upper end of said rotor (3) (i.e. in correspondence of the upper side of the truncated cone 32 that forms the rotor 3) is solidly attached a cylindrical appendage (16); the cylindrical appendage (16) that forms the upper part of the rotor (3) is inclined such that defines a cylindrical projection (16) coaxial to the axis (90) of the rotor (9).
- said stator (1) is provided with an inner cavity (15) conformed and arranged to accommodate said upper cylindrical appendage (16) allowing the latter to rotate within said inner cavity (15).
- the pump (100) comprises a base unit (101) and a stator unit (102) wherein said base unit (101) comprises at least the presser (3) and the rotor (5) and said stator unit (102) comprises at least the stator (1), the tube portion (2), there being provided a base (8) arranged around said rotor (9) not integral with said rotor in rotation and provided with a plate (7) of material suitable for defining a pneumatic seal, there being provided means for determining a lower pressure for associating said base (8) and said stator unit (102) as a seal, resulting in a stable association between said base unit (101) and said stator unit (102).
- said presser (3) comprises an inner shaft (30) provided with an inner core (33) connected to said rotor (9) and an outer truncated cone body (32) defining said presser engagement surface (3), and from the fact that between said inner shaft (30) and said truncated cone body (32) are provided bearings (31) capable of making said truncated cone body (32) rotatable in neutral with respect to the inner shaft (30) and inner core (33).
- said inner shaft (30) comprises an inner core (33) and an outer bushing (34) connected by bearings (31); the inner core (33) is integral with the rotor (9); between the inner core (33) and the outer bushing (34) are provided elastic connecting means (35, 36, 37) configured to allow radial clearance between the two parts.

In general terms, in addition, the invention also relates to an apparatus (200) for veno-venous treatment of blood comprising a pump (100) made according to any of the preceding claims and a disposable kit comprising an oxygenator device (103), wherein said tube portion (2) is provided with an inlet (22) and an outlet (23) and the oxygenator device (103) is provided with an inlet (24) and an outlet (25), the outlet (23) of the tubing portion (2) being connected to the inlet (24) of the oxygenator device (103), and where the inlet (22) of the tubing portion (2) and the outlet (25) of the oxygenator device (103) are provided with means for connection to a patient being treated by connections to the connectors (104, 105) for the input of blood to be treated and the return of treated blood, respectively.

In addition, in the apparatus (200), the pump (100) is equipped with motor means (106) suitable for determining a flow rate between 50 ml/min and 2,000 ml/min. What is described is to be understood with reference to what is illustrated in the attached diagrams that constitute forms of realization of the invention.

In addition, the details of execution can still vary equivalently in shape, size, arrangement of elements, nature of materials used, without, however, leaving the scope of the idea of the solution adopted or the inventive concept and therefore remaining within the limits of the protection granted by the claims that follow.

**Claims**

1.  Peristaltic pump of the type comprising:

    - a portion of tube or circuit-tube (2) intended for the passage of a fluid to be pumped, between an inlet and an outlet, and on which to exert a pressure suitable to generate a direct flow of the fluid from the inlet to the outlet;
    - a stator (1) which is provided with an internal cavity delimited by corresponding walls against which the said portion of tube is pressed;
    - means (17) for fixing said portion of tube (2) to said stator (1);
    - a presser (3) which is moved cyclically along a trajectory to engage said portion of tube (2) by exerting pressure on it through a corresponding engagement surface;
    - means for motorizing said presser element (3) comprising a motorized base or rotor (9) which rotates around an axis (90) and which in its rotation is integral with the presser (3), which is free to rotate around to its axis (30) with respect to said motorized base or rotor (9);
    pump (100) wherein
    - the internal walls of said stator (1) develop along a conical surface having a first aperture angle (A1) and an axis coinciding with the rotation axis (90) of said rotor (9);
    - the engagement surface of the presser (3) is a conical surface having a second aperture angle (A3) different and smaller than said first aperture angle (A1);
    - the presser (3) is supported by a shaft (30) whose axis (30A) does not coincide with the rotation axis (90) of the rotor (9) to which it is integral but inclined with respect to the aforementioned axis, **characterized in that**:

        - the axis (30A) of the presser (3) is inclined with respect to the axis (90) of the rotor (9) by an angle equal to half the difference between said first aperture angle (A1) of the stator and said second aperture angle (A3) of the presser and **in that** the rotation axis (90) of the rotor (9), the axis of the conical surface of the walls inside the stator (90), the presser axis (30A) and the axis of the conical surface to which the presser surface (30A) belongs are all intersecting at a common point (A).

2. Pump according to claim 1, **characterized in that** a cylindrical appendix (16) is fixed integrally to the upper distal end of said internal shaft (33), inclined such that it has, on the opposite plane to that of fixing to the shaft and perpendicular to the axis (90), a cylindrical protrusion (16) coaxial to said axis (90), and **in that** said stator (1) is provided with an internal cavity (15) shaped and arranged so as to accommodate said upper cylindrical appendix (16) allowing the rotation of the latter inside said internal cavity (15).

3. Pump according to one of the previous claims, **characterized in that** the pump (100) is composed of a base unit (101) and a stator unit (102) wherein said base unit (101) comprises at least the presser (3) and the rotor (5) and said stator unit (102) includes at least the stator (1), the tube portion (2), a base (8) being provided arranged around said rotor (9) not integral with the latter in rotation and provided with a plate (7) made of material suitable for defining a pneumatic seal, means being provided for determining a lower pressure to seally associate said base (8) and said stator unit (102), resulting in a stable association between the base unit (101) and the stator unit (102).

4. Pump according to one of the previous claims, **characterized in that** said presser (30) includes an internal shaft (30) provided with an internal core (33) connected to said rotor (9) and an external frusto-conical body (32) which defines said engagement surface of the presser (3) and **in that** between said internal shaft (30) and said frusto-conical body (32) there are bearings (31) suitable for making said frusto-conical body (32) freely rotatable with respect to the internal shaft (30) and to the inner core (33).

5. Pump according to claim 4, **characterized in that** said internal shaft (30) is formed by an internal core (33), integral with the rotor (9), connected to the intermediate shaft (30) and by an external bushing (34), interposed between the internal core (33) and the bearings (31), and **in that** between the internal core (33) and the external bushing (34) there are elastic connection means (35, 36, 37) configured to allow radial clearance between the two parts.

6. Apparatus (200) for the veno-venous treatment of blood, **characterized in that** it includes a pump (100) made according to one of the previous claims and a disposable kit comprising an oxygenator device (103), in which the said tube portion (2) is provided with an inlet (22) and an outlet (23) and the oxygenator device (103) is provided with an inlet (24) and an outlet (25), the outlet (23) of the tube portion (2) being connected to the inlet (24) of the oxygenator device (103), and wherein the inlet (22) of the tube portion (2) and the outlet (25) of the oxygenator (103) are provided with means for connecting to a patient being treated via connections to the connectors (104, 105) respectively for the entry of the blood to be treated and the return of the treated blood.

7. Apparatus (200) according to claim 6, **characterized in that** said pump (100) is provided with motorization means (106) suitable for determining a flow between 50 ml/min and 2,000 ml/min.

**Patentansprüche**

1. Peristaltische Pumpe des Typs, umfassend:

   - einen Abschnitt von Schlauch oder Kreislaufschlauch (2), der für den Durchgang eines zu pumpenden Fluids zwischen einem Einlass und einem Auslass gedacht ist und auf den ein Druck auszuüben ist, der dazu geeignet ist, einen direkten Fluss des Fluids von dem Einlass zu dem Auslass zu erzeugen;
   - einen Stator (1), der mit einem Innenhohlraum bereitgestellt ist, der durch entsprechende Wände begrenzt ist, gegen die der Abschnitt von Schlauch gedrückt wird;
   - Mittel (17) zum Fixieren des Abschnittes von Schlauch (2) an dem Stator (1);
   - einen Drücker (3), der zyklisch entlang einer Bahn bewegt wird, um den Abschnitt von Schlauch (2) in Eingriff zu nehmen, indem Druck auf ihn durch eine entsprechende Eingriffsfläche ausgeübt wird;
   - Mittel zum Motorisieren des Drückerelements (3), umfassend eine motorisierte Basis oder einen motorisierten Rotor (9), die/der sich um eine Achse (90) dreht und die/der bei ihrer/seiner Drehung einstückig mit dem Drücker (3) ist, der sich frei um seine Achse (30) in Bezug auf die motorisierte Basis oder den motorisierten Rotor (9) drehen kann;

   Pumpe (100), wobei

- sich die Innenwände des Stators (1) entlang einer konischen Fläche mit einem ersten Öffnungswinkel (A1) und einer Achse, die mit der Drehachse (90) des Rotors (9) zusammenfällt, entwickeln;
- die Eingriffsfläche des Drückers (3) eine konische Fläche ist, die einen zweiten Öffnungswinkel (A3) aufweist, der sich von dem ersten Öffnungswinkel (A1) unterscheidet und kleiner als dieser ist;
- der Drücker (3) durch eine Welle (30) gestützt wird, deren Achse (30A) nicht mit der Drehachse (90) des Rotors (9) zusammenfällt, zu der er einstückig ist, sondern geneigt in Bezug auf die oben genannte Achse,

**dadurch gekennzeichnet, dass**:

- die Achse (30A) des Drückers (3) in Bezug auf die Achse (90) des Rotors (9) um einen Winkel geneigt ist, der gleich der Hälfte der Differenz zwischen dem ersten Öffnungswinkel (A1) des Stators und dem zweiten Öffnungswinkel (A3) des Drückers ist, und dass sich die Drehachse (90) des Rotors (9), die Achse der konischen Fläche der Wände innerhalb des Stators (90), die Drückerachse (30A) und die Achse der konischen Fläche, zu der die Drückerfläche (30A) gehört, alle in einem gemeinsamen Punkt (A) schneiden.

2. Pumpe nach Anspruch 1, **dadurch gekennzeichnet, dass** ein zylindrischer Ansatz (16) einstückig an dem oberen distalen Ende der Innenwelle (33) befestigt ist, der geneigt ist, sodass er auf der gegenüberliegenden Ebene zu derjenigen der Befestigung an der Welle und senkrecht zu der Achse (90) einen zylindrischen Vorsprung (16) koaxial zu der Achse (90) aufweist, und dass der Stator (1) mit einem Innenhohlraum (15) bereitgestellt ist, der geformt und angeordnet ist, um den oberen zylindrischen Ansatz (16) aufnehmen, der die Drehung des letzteren innerhalb des Innenhohlraums (15) ermöglicht.

3. Pumpe nach einem der vorhergehenden Ansprüche, **dadurch gekennzeichnet, dass** die Pumpe (100) aus einer Basiseinheit (101) und einer Statoreinheit (102) besteht, wobei die Basiseinheit (101) zumindest den Drücker (3) und den Rotor (5) umfasst und die Statoreinheit (102) zumindest den Stator (1), den Schlauchabschnitt (2) beinhaltet, wobei eine Basis (8) angeordnet um den Rotor (9) bereitgestellt ist, nicht einstückig mit dem letzteren in Drehung und mit einer Platte (7) bereitgestellt, die aus Material hergestellt ist, das zum Definieren einer pneumatischen Dichtung geeignet ist, wobei Mittel zum Bestimmen eines niedrigeren Drucks bereitgestellt sind, um die Basis (8) und die Statoreinheit (102) dichtend zu assoziieren, was in einer stabilen Assoziation zwischen der Basiseinheit (101) und der Statoreinheit (102) resultiert.

4. Pumpe nach einem der vorhergehenden Ansprüche, **dadurch gekennzeichnet, dass** der Drücker (30) eine Innenwelle (30) beinhaltet, die mit einem Innenkern (33), der mit dem Rotor (9) verbunden ist, und einem äußeren kegelstumpfförmigen Körper (32) bereitgestellt ist, der die Eingriffsfläche des Drückers (3) definiert, und dass es zwischen der Innenwelle (30) und dem kegelstumpfförmigen Körper (32) Lager (31) gibt, die geeignet sind, um den kegelstumpfförmigen Körper (32) in Bezug auf die Innenwelle (30) und den Innenkern (33) frei drehbar zu machen.

5. Pumpe nach Anspruch 4, **dadurch gekennzeichnet, dass** die Innenwelle (30) durch einen Innenkern (33), der einstückig mit dem Rotor (9) ist, der mit der Zwischenwelle (30) verbunden ist, und durch eine Außenbuchse (34), die zwischen dem Innenkern (33) und den Lagern (31) eingefügt ist, gebildet ist, und dass es zwischen dem Innenkern (33) und der Außenbuchse (34) elastische Verbindungsmittel (35, 36, 37) gibt, die konfiguriert sind, um radiales Spiel zwischen den zwei Teilen zu ermöglichen.

6. Einrichtung (200) für die veno-venöse Behandlung von Blut, **dadurch gekennzeichnet, dass** sie eine Pumpe (100), die nach einem der vorhergehenden Ansprüche hergestellt ist, und ein Einwegkit beinhaltet, das ein Oxygenatorgerät (103) umfasst, in dem der Schlauchabschnitt (2) mit einem Einlass (22) und einem Auslass (23) bereitgestellt ist und das Oxygenatorgerät (103) mit einem Einlass (24) und einem Auslass (25) bereitgestellt ist, wobei der Auslass (23) des Schlauchabschnittes (2) mit dem Einlass (24) des Oxygenatorgeräts (103) verbunden ist und wobei der Einlass (22) des Schlauchabschnittes (2) und der Auslass (25) des Oxygenators (103) mit Mitteln zum Verbinden mit einem behandelten Patienten über Verbindungen mit den Verbindern (104, 105) jeweils für den Eintritt des zu behandelnden Blutes und den Rückfluss des behandelten Blutes bereitgestellt sind.

7. Einrichtung (200) nach Anspruch 6, **dadurch gekennzeichnet, dass** die Pumpe (100) mit Motorisierungsmitteln (106) bereitgestellt ist, die zum Bestimmen eines Flusses zwischen 50 ml/min und 2.000 ml/min geeignet sind.

**Revendications**

1. Pompe péristaltique du type comprenant :

- une portion de tube ou de tube de circuit (2) destinée au passage d'un fluide à pomper, entre une entrée et une sortie, et sur laquelle exercer une pression apte à générer un écoulement direct du fluide de l'entrée vers la sortie ;

- un stator (1) qui est doté d'une cavité interne délimitée par des parois correspondantes contre lesquelles ladite portion de tube est pressée ;

- des moyens (17) pour la fixation de ladite portion de tube (2) audit stator (1) ;

- un presseur (3) qui est déplacé cycliquement le long d'une trajectoire pour venir en prise avec ladite portion de tube (2) en exerçant une pression sur celle-ci par l'intermédiaire d'une surface de mise en prise correspondante ;

- des moyens pour la motorisation dudit élément presseur (3) comprenant une base ou un rotor motorisé(e) (9) qui tourne autour d'un axe (90) et qui, dans sa rotation, est solidaire du presseur (3), qui est libre de tourner autour de son axe (30) par rapport à ladite base ou audit rotor motorisé(e) (9) ;

une pompe (100)

- lesdites parois internes dudit stator (1) se développant le long d'une surface conique possédant un premier angle d'ouverture (A1) et un axe coïncidant avec l'axe de rotation (90) dudit rotor (9) ;

- ladite surface de mise ne prise du presseur (3) étant une surface conique possédant un second angle d'ouverture (A3) différent et plus petit que ledit premier angle d'ouverture (A1) ;

- ledit presseur (3) étant supporté par un arbre (30) dont l'axe (30A) ne coïncide pas avec l'axe de rotation (90) du rotor (9) auquel il est solidaire mais incliné par rapport à l'axe précité, **caractérisée en ce que** :

- l'axe (30A) du presseur (3) est incliné par rapport à l'axe (90) du rotor (9) d'un angle égal à la moitié de la différence entre ledit premier angle d'ouverture (A1) du stator et ledit second angle d'ouverture (A3) du presseur et **en ce que** l'axe de rotation (90) du rotor (9), l'axe de la surface conique des parois à l'intérieur du stator (90), l'axe (30A) de presseur et l'axe de la surface conique à laquelle la surface (30A) de presseur appartient se coupent tous en un point commun (A).

2. Pompe selon la revendication 1, **caractérisée en ce qu'**un appendice cylindrique (16) est fixé d'un seul tenant à l'extrémité distale supérieure dudit arbre interne (33), incliné de sorte qu'il comporte, sur le plan opposé à celui de la fixation à l'arbre et perpendiculaire à l'axe (90), une saillie cylindrique (16) coaxiale audit axe (90), et **en ce que** ledit stator (1) est doté d'une cavité interne (15) façonnée et agencée de façon à recevoir ledit appendice cylindrique supérieur (16) permettant la rotation de ce dernier à l'intérieur de ladite cavité interne (15).

3. Pompe selon l'une des revendications précédentes, **caractérisée en ce que** la pompe (100) est composée d'une unité de base (101) et d'une unité de stator (102), ladite unité de base (101) comprenant au moins le presseur (3) et le rotor (5) et ladite unité de stator (102) comprenant au moins le stator (1), la portion de tube (2), une base (8) prévue agencée autour dudit rotor (9) non solidaire en rotation et dotée d'une plaque (7) constituée d'un matériau approprié pour définir un joint d'étanchéité pneumatique, des moyens étant prévus pour déterminer une pression inférieure pour associer de manière étanche ladite base (8) et ladite unité de stator (102), résultant en une association stable entre l'unité de base (101) et l'unité de stator (102).

4. Pompe selon l'une des revendications précédentes, **caractérisée en ce que** ledit presseur (30) comprend un arbre interne (30) doté d'un noyau interne (33) raccordé audit rotor (9) et un corps tronconique externe (32) qui définit ladite surface de mise en prise du presseur (3) et **en ce qu'**entre ledit arbre interne (30) et ledit corps tronconique (32) se trouvent des paliers (31) adaptés pour rendre ledit corps tronconique (32) librement rotatif par rapport à l'arbre interne (30) et au noyau interne (33).

5. Pompe selon la revendication 4, **caractérisée en ce que** ledit arbre interne (30) est formé par un noyau interne (33), solidaire du rotor (9), raccordé à l'arbre intermédiaire (30) et par une douille externe (34), interposée entre le noyau interne (33) et les paliers (31), et **en ce qu'**entre le noyau interne (33) et la douille externe (34) se trouvent des moyens de raccordement élastiques (35, 36, 37) conçus pour permettre un jeu radial entre les deux pièces.

6. Appareil (200) pour le traitement veino-veineux du sang, **caractérisé en ce qu'**il comprend une pompe (100) réalisée selon l'une des revendications précédentes et un ensemble jetable comprenant un dispositif oxygénateur (103), dans lequel ladite portion de tube (2) est dotée d'une entrée (22) et d'une sortie (23) et le dispositif oxygénateur (103) est doté d'une entrée (24) et d'une sortie (25), la sortie (23) de la portion de tube (2) étant raccordée à l'entrée (24) du dispositif oxygénateur (103), et ladite entrée (22) de la portion de tube (2) et ladite sortie (25) de l'oxygénateur (103) étant dotées de moyens pour le raccordement à un patient en cours de traitement par l'intermédiaire de raccordements aux connecteurs (104, 105) respectivement pour l'entrée du sang à traiter et le retour du sang traité.

7. Appareil (200) selon la revendication 6, **caractérisé en ce que** ladite pompe (100) est dotée de moyens de motorisation (106) adaptés pour déterminer un débit compris entre 50 ml/min et 2 000 ml/min.

FIG. 2

FIG. 3

FIG. 1

FIG. 8

FIG. 9

FIG. 7

FIG. 6

FIG. 5

FIG. 9

FIG. 4

**FIG. 10**

EP 4 610 493 B1

FIG. 11

FIG. 12

**FIG. 13**

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018146541 A
- JP 2001304131 A

- EP 2575926 B1